# EUROPEAN PATENT SPECIFICATION

(11) **EP 2 421 609 B1**
(45) Date of publication and mention of the grant of the patent: **13.02.2013**
(21) Application number: 10721569.1
(22) Date of filing: 22.04.2010
(51) Int. Cl.: A61N 5/06, A61B 17/00, A61B 18/20

(54) **SKIN TREATMENT DEVICE UTILIZING LIGHT AND TEMPERATURE**
VORRICHTUNG ZUR BEHANDLUNG VON HAUT MIT LICHT UND TEMPERATUR
DISPOSITIF DE TRAITEMENT CUTANE UTILISANT LA LUMIERE ET LA TEMPERATURE

(30) Priority: 22.04.2009 US 171476 P
(43) Date of publication of application: 29.02.2012
(73) Proprietor: Radiancy Inc., Orangeburg, NY 10962 (US)
(72) Inventor: SOLOMON, Philip, 99803 Kibbutz Tzora (IL); RAFAELI, Dolev, Cresskill New Jersey 07626 (US); GERTLER, Ifat, 63401 Tel Aviv (IL)
(74) Representative: Greenwood, John David
(86) International application number: PCT/IL2010/000327
(87) International publication number: WO 2010/122561

(56) References cited:
- WO-A1-98/33556
- DE-C1- 19 954 710
- US-A1- 2004 093 042
- US-B1- 6 248 103

## Description

### TECHNICAL FIELD

The invention relates generally to the field of dermatological devices and in particular to a device exhibiting a combination of a temperature adjusting element and a light element arranged to irradiate the target skin.

### BACKGROUND ART

Electromagnetic energy, and particularly light energy in the visible and near infra-red ranges are widely used in medical applications to treat skin disorders. A large range of medical skin conditions, and general aesthetic skin conditions are successfully treated with electromagnetic energy, including but not limited to acne, wrinkle eradication, skin tightening and skin rejuvenation.

U.S. Patent 6,379,376 issued April 30, 2002 to Lubart, the entire contents of which is incorporated herein by reference, is addressed to a method and device for promoting growth and proliferation of skin cells or tissue or for controlling bacterial skin infections. Unfortunately, the technique of Lubart is restricted to the use of light.

U.S. Patent 2008/0300529 published December 4, 2008 to Reinstein, is addressed to a method of treating the skin or body part, comprising contacting the skin or body part with a composition; and contacting the composition and heating and/or cooling the skin or body part with a thermoelectric Peltier device. Unfortunately there is no provision made for phototherapy.

Various products for skin treatment exist which provide a combination of heating and cooling for the skin. The combination is believed to provide various benefits including, but not limited to, skin rejuvenation, reduction of acne and associated inflammations, and improving circulation.

U.S. Patent 7,473,251 issued January 6, 2009 to Knowlton et al, the entire contents of which is incorporated herein by reference, is addressed to a method of creating a tissue effect at a tissue delivery site by delivering electromagnetic energy through a skin surface from an electromagnetic energy delivery device coupled to an electromagnetic energy source.

U.S. Patent 7,351,252 issued April 1, 2008 to Altshuler et al, is addressed to a method and apparatus for treating tissue in a region at depth by applying optical radiation thereto of a wavelength able to reach the depth of the region and of a selected relatively low power for a duration sufficient for the radiation to effect the desired treatment while concurrently cooling tissue above the selected region to protect such tissue. Said document fails to teach alternate operation of light source and temperature adjusting element.

Unfortunately, the prior art does not supply a low cost device providing a user with the combined benefits of both light and temperature therapies.

### SUMMARY OF THE INVENTION

Accordingly, it is an object of the present embodiments to overcome at least some of the disadvantages of prior art devices for skin treatment. This is accomplished in certain embodiments by a hand held, home use, device exhibiting a combination of a light source and a temperature adjusting element, arranged such that irradiation from the light source passes through an aperture in the temperature adjusting element.

In one embodiment the temperature adjusting element is a thermoelectric element. In one embodiment the thermoelectric element is ring shaped, and light energy is provided through an aperture formed in the center opening of the ring shaped thermoelectric element, in another embodiment the thermoelectric element exhibits a matrix of pass-through perforations representing a plurality of apertures and light energy is provided through the plurality of apertures, and in another embodiment the thermoelectric element exhibits a plurality of parallel pass-through slits representing a plurality of apertures and light energy is provided through the plurality of apertures. Preferably, the plurality of apertures are spaced such that the pulsed light energy proceeding from adjacent apertures overlap within the skin treatment area at a predetermined epidermis depth

In one embodiment, the light source is a broad band light source providing light impacting the target area in the range of about 300 - 2000 nm, and in another embodiment the light source is filtered light providing light impacting the target area in the range of 590 - 2000 nm.

In one embodiment the light source is pulsed, the pulses being of a duration such that energy per pulse at the target skin area is 0.05 - 1 J/cm², and preferably 0.3 - 0.6 J/cm². The number of pulses is selected such to provide a fluence at the target skin area over a treatment session of 4 - 25 J/cm², and preferably a fluence at the target skin area over a treatment session of 8 - 12 J/cm².

In one embodiment the temperature adjusting element operates in a cooling mode and provides for a temperature of 0 - 25° C in contact with the user skin, preferably 4 - 15° C. In one embodiment cold and light are alternately pulsed.

In one embodiment, the portion of the skin treatment area is in the range of 0.25 - 2 cm². In another embodiment the portion of the skin treatment area is in the range of 0.5 - 1 cm².

In one embodiment the pulse train of the light source exhibits a frequency of 0.1 - 10 Hz and a duty cycle of no more than 50%. In another embodiment the pulse train of the light source exhibits a frequency of 0.25 - 5 Hz and a duty cycle of no more than 50%.

In one particular embodiment, a hand held device for treatment of a skin treatment area is provided, the device comprising: a housing exhibiting an opening therein; a temperature adjusting element secured to an end of the housing, one end of the temperature adjusting element arranged to contact the skin treatment area, the temperature adjusting element exhibiting at least one aperture passing there through; a light source secured to the housing; a light path arranged to pass light energy from the light source to at least a portion of the skin treatment area via the at least one aperture; and a control and driving circuitry in electrical communication with each of the light source and the temperature adjusting element, the control and driving circuitry operative to alternately : output a train of pulses to the light source thereby providing pulsed light energy to the portion of the skin treatment area from the light source proceeding through the aperture; and power the temperature adjusting element so as to adjust the temperature of the skin treatment area.

Also described is a method of treating skin , the method comprising: applying a temperature adjusting surface to a skin treatment area; providing at least one aperture in the applied temperature adjusting surface; and providing pulsed light energy to a portion of the skin treatment area via the provided aperture.

In one further embodiment the temperature adjusting surface is a cooling surface exhibiting a temperature of less than 25°C to the skin treatment area. The method may further comprise : pulsing the temperature adjusting element alternately with the pulsed light energy.

In one further embodiment at least one aperture comprises a plurality of apertures spaced such that the pulsed light energy proceeding from adjacent apertures overlap within the skin treatment area at a predetermined epidermis depth. In another further embodiment the pulsed light energy provides a fluence of 4 - 25 J/cm² at the portion of the skin treatment area over a predetermined treatment time.

In one further embodiment pulsed light energy provides a fluence of 8 - 12 J/cm² at the portion of the skin treatment area over a predetermined treatment time. In another further embodiment the predetermined treatment time is in the range of 5 - 60 seconds.

In one further embodiment the predetermined treatment time is in the range of 25 - 35 seconds. In another further embodiment each of the pulses of the pulsed light energy provides a fluence of 0.05 - 1 J/cm² of light energy at the portion of the skin treatment area.

In one further embodiment each of the pulses of the pulsed light energy provides a fluence of 0.3 - 0.6 J/cm² of light energy at the portion of the skin treatment area. In another further embodiment the provided pulsed light energy exhibits wavelengths in the range of 300 - 2000 nm, preferably in the range of 590 - 2000 nm.

In one further embodiment the portion of the skin treatment area is in the range of 0.25 - 2 cm², preferably in the range of 0.5 - 1 cm². In another further embodiment the pulsed light energy exhibits a frequency of 0.1 - 10 Hz and a duty cycle of no more than 50%, preferably the pulsed light energy exhibits a frequency of 0.25 - 5 Hz and a duty cycle of no more than 50%.

Additional features and advantages will become apparent from the following drawings and description.

### BRIEF DESCRIPTION OF THE DRAWINGS

For a better understanding of the invention and to show how the same may be carried into effect, reference will now be made, purely by way of example, to the accompanying drawings in which like numerals designate corresponding elements or sections throughout.

With specific reference now to the drawings in detail, it is stressed that the particulars shown are by way of example and for purposes of illustrative discussion of the preferred embodiments of the present invention only, and are presented in the cause of providing what is believed to be the most useful and readily understood description of the principles and conceptual aspects of the invention. In this regard, no attempt is made to show structural details of the invention in more detail than is necessary for a fundamental understanding of the invention, the description taken with the drawings making apparent to those skilled in the art how the several forms of the invention may be embodied in practice. In the accompanying drawings:

Fig. 1A illustrates a high level schematic diagram of a perspective view of a hand held device in accordance with certain embodiments;

Fig. 1B illustrates a high level schematic diagram of a side cut view of the hand held device of Fig. 1A;

Fig. 1C illustrates a high level schematic diagram of a skin treatment area for treatment with the hand held device of Figs. 1A, 1B;

Fig. 1D illustrates a perspective view of an embodiment of a temperature adjusting element exhibiting a central single aperture in accordance with certain embodiments;

Fig. 1E illustrates a perspective view of an embodiment of a temperature adjusting element exhibiting a matrix of substantially cylindrical apertures in accordance with certain embodiments;

Fig. 1F illustrates a perspective view of an embodiment of a temperature adjusting element exhibiting a matrix of substantially box shaped apertures in accordance with certain embodiments;

Fig. 1G illustrates a perspective view of an embodiment of a temperature adjusting element exhibiting a plurality of parallel slit shaped apertures in accordance with certain embodiments;

Fig. 1H illustrates a side view of a skin treatment portion showing an overlap of radiation within the epidermis in accordance with certain embodiments;

Fig. 2 illustrates a high level block diagram of the hand held device of Figs. 1A and 1B in accordance with certain embodiments;

Fig. 3 illustrates a high level schematic diagram in greater detail of the circuitry of hand held device of Figs. 1A,1B and 2 in accordance with certain embodiments;

Fig. 4 illustrates a graph of the power output of a light source during a treatment session in accordance with certain embodiments;

Fig. 5 illustrates a graph of the surface temperature of a user skin during a treatment session; and

Fig. 6 illustrates a high level flow chart of a method of skin treatment.

### DETAILED DESCRIPTION OF THE PREFERRED EMBODIMENTS

Before explaining at least one embodiment of the invention in detail, it is to be understood that the invention is not limited in its application to the details of construction and the arrangement of the components set forth in the following description or illustrated in the drawings. The invention is applicable to other embodiments or of being practiced or carried out in various ways. Also, it is to be understood that the phraseology and terminology employed herein is for the purpose of description and should not be regarded as limiting.

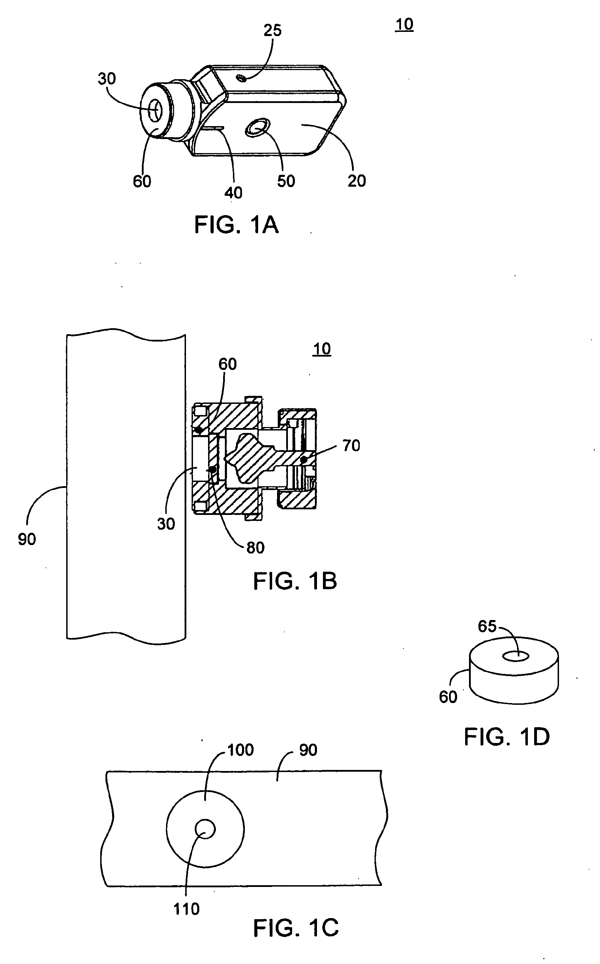

Fig. 1A illustrates a high level schematic diagram of a perspective view of a hand held device 10, Fig. 1B illustrates a high level schematic diagram of a side cut view of hand held device 10, Fig. 1C illustrates a high level schematic diagram of a skin treatment area of hand held device 10 in accordance with certain embodiments, and Fig. 1D illustrates a perspective view of an embodiment of a temperature adjusting element in accordance with certain embodiments, Figs. 1A - 1D being taken together. Hand held device 10 comprises: a housing 20; a charger jack 25; a light path 30; a visual indicator 40; a user input device 50; a temperature adjusting element 60 exhibiting an aperture 65; a light source 70; an optional optical filter 80; a skin area 90 exhibiting a skin treatment area 100 and a skin treatment area portion 110.

Visual indicator 40 preferably comprises one or more LEDs or an LCD display. User input device 50 is preferably a push button, and is illustrated as such, however this is not meant to be limiting in any way. In one embodiment light source 70 comprises an incandescent type bulb. In one embodiment, temperature adjusting element 60 is implemented as a thermoelectric device operative responsive to the Peltier effect, one temperature controlled side of which is arranged to contact skin area 90. Temperature adjusting element 60 is arranged for placement in contact with skin area 90 and the portion of skin area 90 in communication with temperature adjusting element 60 defines skin treatment area 100. The portion of skin area 90 irradiated by light proceeding from light source 70 via light path 30 and proceeding through aperture 65 defines skin treatment area portion 110. In one non-limiting embodiment skin treatment area 100 is on the order of 3 - 6 cm². In one embodiment skin treatment area portion 110 is in the range of 0.25 - 2 cm², and preferably in the range of 0.5 -1 cm².

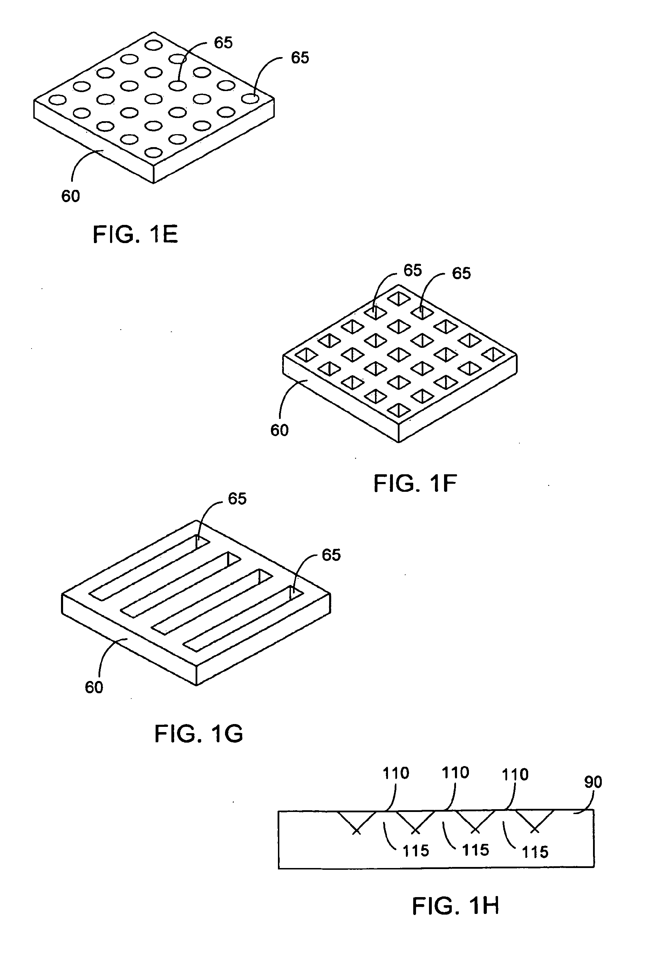

Temperature adjusting element 60 is illustrated as a ring shaped element, with light path 30 proceeding via central aperture 65 of ring shaped temperature adjusting element 60, however this is not meant to be limiting in any way, and light path 30 and temperature adjusting element 60 may be provided as a matrix of apertures or a group of alternating parallel stripes, as described in relation to Figs. 1E - 1G, or any other geometric combination.

Light source 70 outputs light energy, and in one embodiment the light energy output exhibits wavelengths in the range of 300 - 2000 nm. The light energy proceeding from light source 70 along light path 30 is optionally filtered by optional optical filter 80 to remove shorter wavelengths, and thus irradiate skin treatment area portion 110 with light energy exhibiting wavelengths in the range of 590 - 2000 nm. Longer wavelengths are optionally preferred as they provide deeper skin penetration.

Charger jack 25 provides for charging of an on-board rechargeable battery. Optionally, hand held device 10 is battery operated, or mains operated, without exceeding the scope.

In operation, and as will be described further hereinto below, responsive to a user input at user input device 50, skin treatment area portion 110 is irradiated with light energy proceeding from light source 70, preferably pulsed light energy, preferably with a frequency of 0.1 - 10 Hz and further preferably with a frequency of 0.25 - 5 Hz, for a total treatment time of 5 - 60 seconds, and preferably 25 - 35 seconds. The pulsed light energy exhibits a fluence over the total treatment time, defined at skin treatment area portion 110, of 4 - 25 J/cm², and preferably 8 - 12 J/cm², with an energy per pulse of preferably 0.05 - 1 J/cm² and further preferably 0.3 - 0.6 J/cm². In one embodiment, temperature adjusting element 60 is further powered to provide cooling of skin treatment area 100 over the total treatment time, and preferably the cooling is powered alternately with the pulsed light energy. In one embodiment the cooling temperature of temperature adjusting element 60 is 0 - 25°C, and preferably 4 - 15°C. In another embodiment, temperature adjusting element 60 is further powered to provide heating of skin treatment area 100 over the total treatment time, and preferably the heating is powered alternately with the pulsed light energy.

Fig. 1E illustrates a perspective view of an embodiment of a temperature adjusting element 60 exhibiting a matrix of substantially cylindrical apertures 65 in accordance with certain embodiments. Optionally apertures 65 are sufficiently closely spaced so that the light energy from light source 70, and particularly infra-red radiation, overlap at a predetermined depth of the epidermis of skin treatment area portion 110, as illustrated in Fig. 1H, wherein a skin treatment area depth 115 is shown wherein the radiation area expands with skin depth. Temperature element 60 of Fig. 1E is illustrated as a rectangular element, however this is not meant to be limiting in any way, and temperature element 60 may be implemented as a cylindrical shaped element or any other shape, without limitation, without exceeding the scope.

Fig. 1F illustrates a perspective view of an embodiment of a temperature adjusting element 60 exhibiting a matrix of substantially box shaped apertures 65 in accordance with certain embodiments. Optionally apertures 65 are sufficiently closely spaced so that the light energy from light source 70, and particularly infra-red radiation, overlap at a predetermined depth of the epidermis of skin treatment area portion 110, as illustrated in Fig. 1H, wherein a skin treatment area depth 115 is shown wherein the radiation area expands with skin depth. Temperature element 60 of Fig. 1F is illustrated as a rectangular element, however this is not meant to be limiting in any way, and temperature element 60 may be implemented as a cylindrical shaped element or any other shape, without limitation, without exceeding the scope.

Fig. 1G illustrates a perspective view of an embodiment of a temperature adjusting element 60 exhibiting a plurality of parallel slit shaped apertures 65 in accordance with certain embodiments. Optionally, parallel slit shaped apertures 65 are sufficiently closely spaced so that the light energy from light source 70, and particularly infra-red radiation, overlap at a predetermined depth of the epidermis of skin treatment area portion 110, as illustrated in Fig. 1H, wherein a skin treatment area depth 115 is shown wherein the radiation area expands with skin depth. Temperature element 60 of Fig. 1G is illustrated as a rectangular element, however this is not meant to be limiting in any way, and temperature element 60 may be implemented as a cylindrical shaped element or any other shape, without limitation, without exceeding the scope.

The alternating light and temperature therapy is believed to increase blood flow to skin treatment area 100, and particularly skin treatment area portion 110, thus increasing metabolism of the constituent cells of skin treatment area 100.

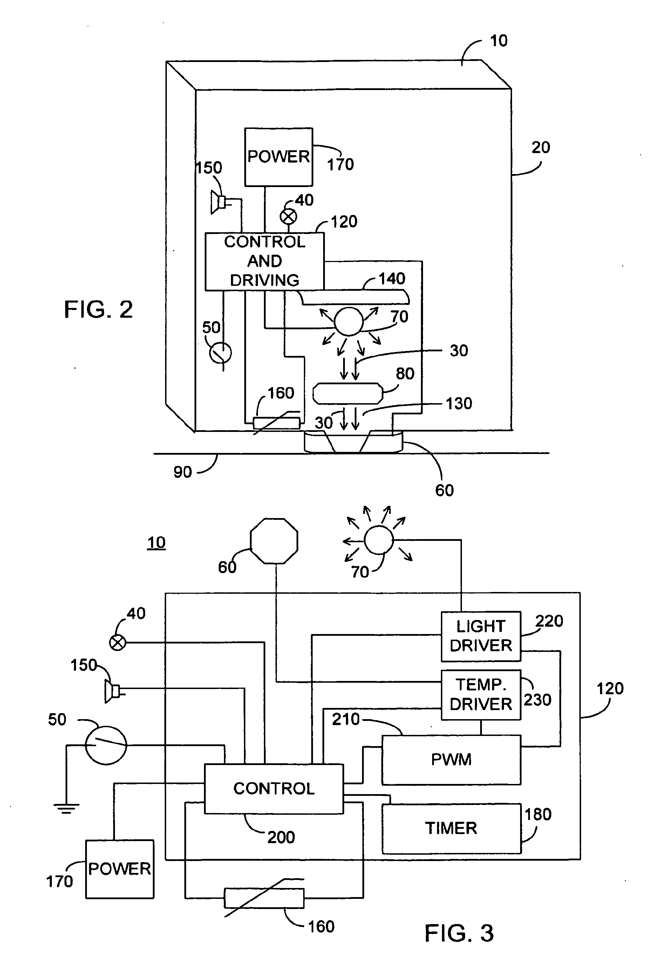

Fig. 2 illustrates a high level block diagram of hand held device 10 of Figs. 1A and 1B in accordance with certain embodiments comprising: housing 20 exhibiting a light path 30 arranged for placement in contact with skin treatment area portion 110; a light source 70; a control and driving circuitry 120; a reflector 140; an audible alarm 150; a visual indicator 40; a user input device 50; a temperature sensor 160; a rechargeable power source 170; a temperature adjusting element 60; an optional optical filter 80; and an optional air cavity 130. Control and driving circuitry 120 is in communication with each of: visual indicator 40; user input device 50; temperature sensor 160; rechargeable power source 170; light source 70; and temperature adjusting element 60. Light path 30 is defined by an optical channel between light source 70 and skin treatment area portion 110, and in an exemplary embodiment, as described above, proceeds through one or more apertures 65 in temperature adjusting element 60. Optional optical filter 80 is disposed within light path 30, and is operative to filter out unwanted wavelengths output by light source 70, preferably wavelengths of less than 590 nm. Optional air cavity 130 is formed between light source 70, or between optional optical filter 80 if supplied, and skin treatment area portion 110, and further supplies heat to skin treatment area portion 110. In one embodiment the heat for optional air cavity 130 is supplied by light source 70, and a temperature gradient is formed between light source 70 and skin treatment area portion 110. In another embodiment a separate heating element (not shown) is further supplied. In yet another embodiment, in which temperature adjusting element 60 is implemented as a thermoelectric element, and the skin is cooled by temperature adjusting element 60, the opposing heated side provides heat to optional air cavity 130. In an embodiment in which optional optical filter 80 is supplied, and arranged to be in contact with skin treatment area portion 110, optional air cavity 130 is not present. Temperature sensor 160 is arranged to sense a temperature associated with the temperature of one of skin treatment area 100 and skin treatment area portion 110.

In operation, control and driving circuitry 120 senses a user input via user input device 50. Responsive thereto, control and driving circuitry 120 is operative to energize visual indicator 40, which preferably comprises one or more LEDs or an LCD display, thus indicating operation to the user. Visual indicator 40 may be further operative to output additional status indication, such as a charging status of rechargeable power source 170, operation of light source 70, and/or a temperature range of one of skin treatment area 100 and skin treatment area portion 110 responsive to the output of temperature sensor 160. Audible alarm 150, which in one embodiment is constituted of a buzzer, is operative to audibly notify a user of the end of the treatment session, or alternatively of a failure condition of any of light source 70 and temperature adjusting element 60, or an out of temperature range condition. Control and driving circuitry 120 is further preferably operative to adjust one or more of the PWM duty cycle of temperature adjusting element 60, the PWM duty cycle of light source 70, the power per cycle applied to temperature adjusting element 60 and the power per cycle applied to light source 70 responsive to temperature sensor 160, thus ensuring that the temperature of one of skin treatment area 100 and skin treatment area portion 110 remains within predetermined parameters. In one non-limiting embodiment, a plurality of different treatment programs are user selectable via user input device 50, such as a low energy program, a moderate energy program and a maximum energy program, each of which programs provide energy in a different powering range.

Light source 70 is secured in relation to housing 20, and preferably secured within housing 20, and receives pulsed power from control and driving circuitry 120 exhibiting an on time during which a current is driven through light source 70 and an off time during which current is not driven through light source 70. Preferably, the pulsed power exhibits a duty cycle of up to 50%. Reflector 140 is disposed within housing 20 and is arranged to reflect light exiting light source 70 towards light path 30. In one non-limiting embodiment, light source 70 is constituted of an incandescent or halogen type bulb.

As described above, temperature adjusting element 60 is in one non-limiting embodiment a thermo-electric element working responsive to the principle of the Peltier effect. In another non-limiting embodiment temperature adjusting element 60 comprises at least one of a gas, liquid and solid, or a plurality thereof, normally used for cooling. As described above in relation to Figs. 1A and 1B, temperature adjusting element 60 is preferably arranged to be in contact with skin treatment area 100, and exhibits an aperture 65 for the passage of light energy from light source 70. The area of impact of light energy defines skin treatment area portion 110. Temperature adjusting element 60 is responsive to control and driving circuitry 120 and receives pulsed power there from exhibiting an on time in which temperature adjusting element 60 is operative to cool skin treatment area 100 and an off time when temperature adjusting element 60 does not provide cooling, except for any residual cooling caused by the thermal mass of temperature adjusting element 60. Preferably, temperature adjusting element 60 is pulsed with power alternately with the pulsed energy supplied to light source 70. Thus, when light source 70 outputs light energy, temperature adjusting element 60 is quiescent, and when light source 70 is quiescent, temperature adjusting element 60 is active to cool skin treatment area 100. In one embodiment the cooling temperature of temperature adjusting element 60 is 0 - 25°C, and preferably 4 -15°C.

Control and driving circuitry 120 is connected to rechargeable power source 170, and is operative to monitor the status thereof, control charging thereof and draw power there from.

As indicated above, in another embodiment, temperature adjusting element 60 is responsive to control and driving circuitry 120 and receives pulsed power there from exhibiting an on time in which temperature adjusting element 60 is operative to heat skin treatment area 100 and an off time when temperature adjusting element 60 does not provide heating, except for any residual heating caused by the thermal mass of temperature adjusting element 60.

Fig. 3 illustrates a high level schematic diagram of the circuitry of hand held device 10 of Figs. 1A, 1B and 2 in accordance with certain embodiments, showing in greater detail control and driving circuitry 120. Hand held device 10 comprises: a light source 70; a control and driving circuitry 120; an audible alarm 150; a visual indicator 40; a user input device 50; a temperature sensor 160; a rechargeable power source 170; and a temperature adjusting element 60. Control and driving circuitry 120 comprises: a control block 200; a pulse width modulation (PWM) generator 210; a light source driving circuitry 220; a temperature adjusting element driving circuitry 230; and a timer 180.

Control block 200 is in communication with PWM generator 210, light source driving circuitry 220, temperature adjusting element driving circuitry 230, timer 180, user input device 50, visual indicator 40, audible alarm 150, rechargeable power source 170 and temperature sensor 160. A first output of PWM generator 210 is fed to light source driving circuitry 220, and the output of light source driving circuitry 220 is connected to light source 70. A second output of PWM generator 210 is fed to temperature adjusting element driving circuitry 230, and the output of temperature adjusting element driving circuitry 230 is connected to temperature adjusting element 60.

In operation, control block 200 monitors the status of rechargeable power source 170. In the event that rechargeable power source 170 is connected to an external charging source via charger jack 25, as described above in relation to Fig. 1A, and the voltage of rechargeable power source 170 exceeds a predetermined maximum, charging of rechargeable power source 170 is interrupted.

As described above, responsive to a user action at user input device 50, control block 200 is activated to begin a treatment session. In one embodiment a treatment session is within the range of 5 - 60 seconds, preferably in the range of 25 - 35 seconds. Preferably, control block 200 is operative to set timer 180 to load the value of the predetermined treatment session and to output a signal at the end of the predetermined treatment session. Visual indicator 40 is set to indicate operation. In a preferred embodiment PWM generator 210 is set to produce a pulse train exhibiting a 50% duty cycle. Further preferably, the output of PWM generator 210 connected to light source driving circuitry 220 operates alternately with the output of PWM generator 210 connected to temperature adjusting element driving circuitry 230. Once PWM generator 210 has stabilized, light source driving circuitry 220 and temperature adjusting element driving circuitry 230 are enabled, and timer 180 is initialized. In one embodiment the frequency of PWM generator 210 is set to be 0.1 - 10 Hz, and preferably 0.25 - 5 Hz. In one embodiment the frequency of PWM generator 210 can be adjusted during treatment by control block 200. In another embodiment PWM generator 210 is set to produce a pulse train exhibiting a duty cycle up to 50%. In another embodiment the duty cycle of the pulse train can be adjusted during treatment by control block 200. In one particular embodiment, as described above, the duty cycle is adjusted responsive to an output of temperature sensor 160. In another embodiment PWM generator 210 is operative to produce two independent pulse trains, with light source driving circuitry 220 arranged to drive light source 70 responsive to a first of the two pulse trains and temperature adjusting element driving circuitry 230 arranged to drive temperature adjusting element 60 responsive to a second of the two pulse trains.

The operation of light source driving circuitry 220 and temperature adjusting element driving circuitry 230 is continued until a signal is received from timer 180 indicating that the treatment session has completed. Upon completion of the predetermined treatment session time, light source driving circuitry 220 and temperature adjusting element driving circuitry 230 are each disabled, and preferably an audible indicator is output via audible alarm 150. The overall fluence of the light received at skin treatment area portion 110 over the treatment session is in one embodiment 4 - 25 J/cm², and preferably 8-12 J/cm², and the power and duty cycle of light element driving circuitry 220 is set to achieve the desired fluence. Preferably, the fluence of each light pulse is 0.05 - 1 J/cm², and further preferably 0.3 - 0.6 J/cm². In the event that an out of temperature range is detected by control block 200 responsive to temperature sensor 160, the duty cycle of one or more of light source driving circuitry 220 and temperature adjusting element driving circuitry 230 is preferably adjusted to maintain skin temperature within acceptable parameters.

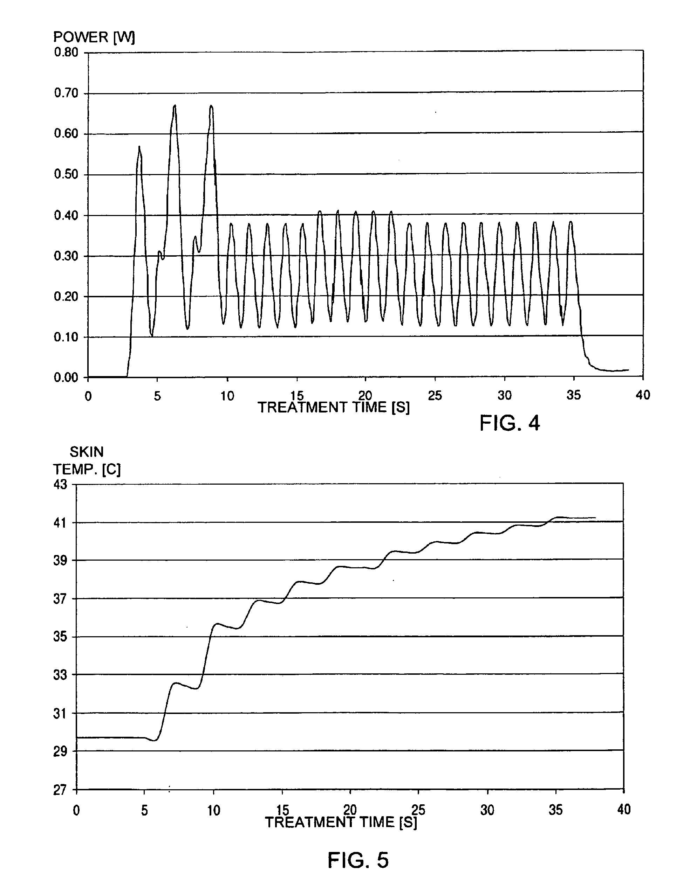

Fig. 4 illustrates a graph of the power output of light source 70 during a treatment session, in which the x-axis represents time in seconds and the y-axis represents power output in Watts measured at skin treatment area portion 110 of Fig. 1C. In one embodiment, the initial pulses are of greater intensity and width so as to achieve greater absorption by the target area skin portion, since the skin absorbs energy more efficiently at the beginning of the treatment due to the initial low temperature of the skin. During treatment, as described above, PWM generator 210 produces a pulse train, optionally with an adjustable duty rate, thereby enabling light source driving circuitry 220 to drive light source 70. In a preferred embodiment, as described above, light source 70 and temperature adjusting element 60 are driven alternately, with temperature adjusting element 60 being driven by temperature adjusting element driving circuitry 230 when light source driving circuitry 220 is not driving light source 70.

Fig. 5 illustrates a graph of the surface temperature of skin treatment area portion 110 of Fig. 1C during treatment, where temperature adjusting element 60 is operated in a cooling mode, in which the x-axis represents time in seconds and the y-axis represents surface skin temperature in degrees centigrade. As described above in relation to Fig. 4, at the beginning of a treatment light source 70 is in one embodiment driven with long pulses. This results in relatively large rises in the surface temperature of skin treatment area portion 110 as it is irradiated for relatively long periods, and because the skin absorbs energy more efficiently at the beginning of the treatment due to the initial low temperature of the skin, as described above. After the initial long pulses, light source 70 is driven with shorter pulses, thereby resulting in smaller rises of the surface temperature of skin treatment area portion 110. As described above temperature adjusting element 60 is driven, preferably during the periods when light source 70 is not driven, thereby cooling the surface temperature of skin treatment area 100, and in particular skin treatment area portion 110. The alternating pulses of cooling power and irradiation results in a more effective treatment, particularly believed to increase blood flow and as a result cell metabolism. As shown, after each rise in the surface temperature of skin treatment area portion 110, temperature adjusting element 60 causes a decrease in the surface temperature of skin treatment area portion 110 thereby tightening skin treatment area portion 110 and resulting in more effective treatment.

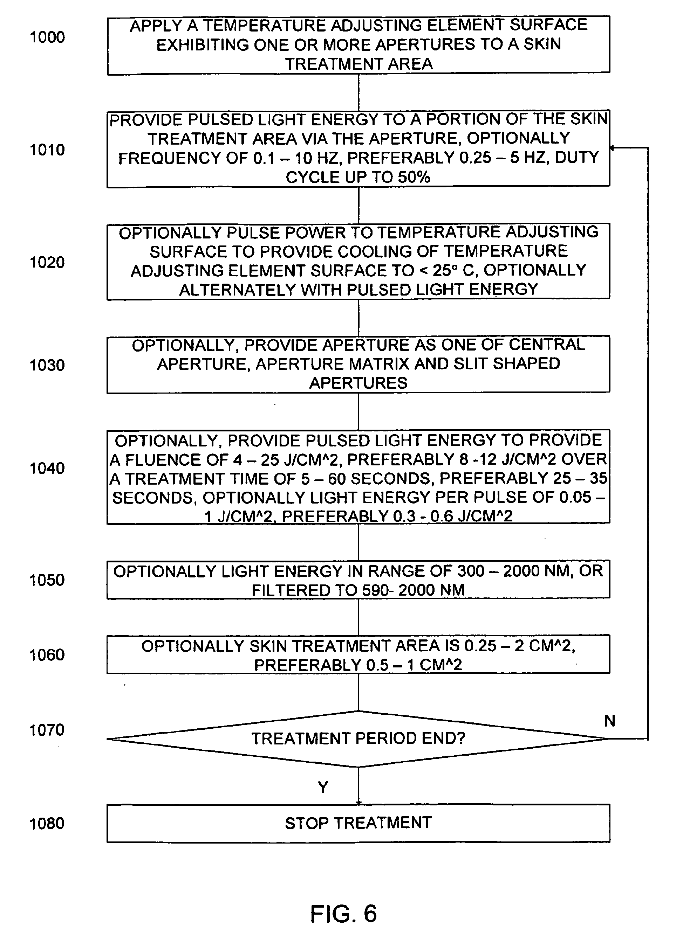

Fig. 6 illustrates a high level flow chart of a method of skin treatment where the temperature adjusting element is used in a cooling mode. In stage 1000, a temperature adjusting surface such as temperature adjusting element 60 described above, is applied to a skin treatment area, such as skin treatment area 100 described above, the temperature adjusting surface being provided with one or more apertures. In stage 1010, a pulsed light energy is applied to a portion of the skin treatment area of stage 1000 via a light path proceeding through the one or more apertures of stage 1000. Optionally, the light energy is pulsed at a frequency of 0.1 - 10 Hz, and preferably 0.25 - 5 Hz, with a duty cycle of up to 50%. In optional stage 1020 the temperature adjusting element is powered in the cooling mode, providing cooling to the skin treatment area, by a pulsed power, exhibiting a temperature of less than 25° C, preferably alternately with the pulsed power of stage 1010. There is no requirement that stage 1010 precedes stage 1020, and stage 1020 may precede stage 1010 without exceeding the scope.

In optional stage 1030, the one or more apertures of stage 1000 are provided as a one of a central aperture, a matrix of apertures and a plurality of parallel slit shaped apertures.

In optional stage 1040, the pulsed light energy of stage 1010 is arranged to provide a fluence in the range of 4 - 25 J/cm², and preferably 8-12 J/cm², over a treatment session time predetermined to be preferably in the range of 5 - 60 seconds, and further preferably in the range of 25 - 35 seconds. Optionally, the energy per pulse is set to 0.05 - 1 J/cm² and preferably 0.3- 0.6 J/cm².

In optional stage 1050, the pulsed light energy of stage 1010 is arranged to be a broadband light source exhibiting wavelengths in the range of 300 - 2000 nm, and further optionally the light is filtered to exhibit wavelengths in the range of 590 of 2000 nm.

In optional stage 1060, the skin treatment area portion of stage 1010 is set to be in the range of 0.25 - 2 cm², and preferably 0.5 - 1 cm².

In stage 1070 the treatment period time is monitored. In one non-limiting embodiment, the treatment period time is monitored by checking an input from timer 180 of Fig. 3. In the event that the treatment period has ended, in stage 1080, treatment is stopped, preferably by disabling stages 1010, 1020. In one embodiment an audible and/or visual warning is further supplied to the user. In the event that in stage 1070 the treatment period has not ended, stage 1010 as described above is again performed.

Thus, certain of the present embodiments enable a hand held, home use, device exhibiting a combination of a light source and a temperature adjusting element exhibiting an aperture. In one embodiment the temperature adjusting element is a thermoelectric element provided with one of a central aperture, a matrix of apertures or a plurality of parallel slit shaped apertures, and light energy is provided through the aperture to the skin. In one embodiment, the light source is a broad band light source providing light impacting the target area in the range of about 300 - 2000 nm, and in another embodiment the light source is filtered light providing light impacting the target area in the range of 590 - 2000 nm.

In one embodiment the light source is pulsed, the pulses being of a duration such that energy per pulse at the target skin area is 0.05 - 1 J/cm², and preferably 0.3-0.6 J/cm². The number of pulses is selected such to provide a fluence at the target skin area over a treatment session of 4 - 25 J/cm², and preferably a fluence at the target skin area over a treatment session of 8 -12 J/cm².

In one embodiment the temperature adjusting element in a cooling mode provides for a temperature of 0 - 25° C in contact with the user skin, preferably 4 - 15° C. In one embodiment cold and light are alternately pulsed.

It is appreciated that certain features of the invention, which are, for clarity, described in the context of separate embodiments, may also be provided in combination in a single embodiment. Conversely, various features of the invention which are, for brevity, described in the context of a single embodiment, may also be provided separately or in any suitable sub-combination. In the claims of this application and in the description of the invention, except where the context requires otherwise due to express language or necessary implication, the word "comprise" or variations such as "comprises" or "comprising" is used in any inclusive sense, i.e. to specify the presence of the stated features but not to preclude the presence or addition of further features in various embodiments of the invention.

Unless otherwise defined, all technical and scientific terms used herein have the same meanings as are commonly understood by one of ordinary skill in the art to which this invention belongs.

The materials, methods, and examples are illustrative only and not intended to be limiting.

It will be appreciated by persons skilled in the art that the present invention is not limited to what has been particularly shown and described hereinabove. Rather the scope of the present invention is defined by the appended claims and includes both combinations and sub-combinations of the various features described hereinabove as well as variations and modifications thereof, which would occur to persons skilled in the art upon reading the foregoing description.

## Claims

1. A hand held device (10) for treatment of a skin treatment area, the device (10) comprising:
a housing (20) exhibiting an opening therein;
a temperature adjusting element (60) secured to an end of said housing (20), one end of said temperature adjusting element (60) arranged to contact the skin treatment area, said temperature adjusting element (60) exhibiting at least one aperture (65) passing there through;
a light source (70) secured to said housing (20);
a light path (30) arranged to pass light energy from said light source (70) to at least a portion of the skin treatment area (110) via said at least one aperture (65); and
a control and driving circuitry (120) in electrical communication with each of said light source (70) and said temperature adjusting element (60), said control and driving circuitry operative to alternately:
output a train of pulses to said light source (70) thereby providing pulsed light energy to said portion of the skin treatment area (110) from said light source (70) proceeding through said aperture (65); and
power said temperature adjusting element (60) so as to adjust the temperature of the skin treatment area (100).

2. A hand held device (10) according to claim 1, wherein said temperature adjusting element (60) is a thermoelectric element.

3. A hand held device (10) according to claim 1 or claim 2, wherein said temperature adjusting element (60) is arranged to provide a temperature of less than 25°C at said one end arranged to contact the skin treatment area (100).

4. A hand held device (10) according to claim 1, wherein said control and driving circuitry (120) is operative to power said temperature adjusting element (60) with a train of pulses.

5. A hand held device (10) according to claim 1, wherein said temperature adjusting element (60) is ring shaped, and said aperture (65) represents a central opening in said ring shape (60).

6. A hand held device (10) according to claim 1, wherein said at least one aperture (65) comprises one of a matrix of apertures and a plurality of slit shaped apertures.

7. A hand held device (10) according to claim 6, wherein said plurality of apertures are spaced such that said pulsed light energy proceeding from adjacent apertures overlap within said skin treatment area (100) at a predetermined epidermis depth (115).

8. A hand held device (10) according to claim 1, wherein said train of pulses of said light source (70) are of sufficient energy to provide a fluence of 4 - 25 J/cm² of light energy from said light source (70) at said portion of the skin treatment area (110) over a predetermined treatment time.

9. A hand held device (10) according to claim 8, wherein the predetermined treatment time is in the range of 5 - 60 seconds.

10. A hand held device (10) according to claim 1, wherein each of said pulses of said train of pulses are of sufficient energy to provide a fluence of 0.05 - 1 J/cm² of light energy from said light source (70) at said portion of the skin treatment area (110).

11. A hand held device (10) according to claim 10, wherein each of said pulses of said train of pulses are of sufficient energy to provide a fluence of 0.3 - 0.6 J/cm² of light energy from said light source (70) at said portion of the skin treatment area (110).

12. A hand held device (10) according to claim 1, wherein said light source (70) outputs light exhibiting wavelengths in the range of 300 - 2000 nm.

13. A hand held device (10) according to claim 1, wherein said portion of the skin treatment area (110) is in the range of 0.25 - 2 cm².

14. A hand held device (10) according to claim 1, wherein said pulse train of said light source exhibits a frequency of 0.1 - 10 Hz and a duty cycle of no more than 50%.

15. A hand held device (10) according to claim 1, wherein said pulse train of said light source exhibits a frequency of 0.25 - 5 Hz and a duty cycle of no more than 50%.

## Patentansprüche

1. Ein Handgerät (10) zur Behandlung eines Behandlungsbereichs der Haut, bestehend aus:
- einem Gehäuse (20) mit einer Öffnung darin;
- einem Temperatureinstellelement (60), das an einem Ende besagten Gehäuses (20) befestigt ist, wobei ein Ende besagten Temperatureinstellelements (60) so angeordnet ist, dass es den Behandlungsbereich der Haut berührt, und besagtes Temperatureinstellelement (60) zumindest eine Öffnung aufweist für:
- eine an besagtem Gehäuse (20) befestigte Lichtquelle (70);
- einen Lichtweg (30), der so angeordnet ist, dass er Lichtenergie von besagter Lichtquelle (70) an mindestens einen Teil des Behandlungsbereichs der Haut (110) über besagte zumindest eine Öffnung (65) leitet; und
- eine Steuer- und Treiberschaltung (120), die elektrisch mit jeweils der besagten Lichtquelle (70) und dem besagten Temperatureinstellelement (60) verbunden ist, wobei besagte Steuer- und Treiberschaltung im Betrieb abwechselnd
- eine Folge von Impulsen an besagte Lichtquelle (70) aussendet und dabei gepulste Lichtenergie auf besagten Teil des Behandlungsbereichs der Haut (110) von besagter Lichtquelle (70) durch besagte Öffnung (65) leitet; und
- besagtes Temperatureinstellelement (60) antreibt, so dass die Temperatur des Behandlungsbereichs der Haut (100) eingestellt wird.

2. Ein Handgerät (10) nach Anspruch 1, wobei es sich bei besagtem Temperatureinstellelement (60) um ein thermoelektrisches Element handelt.

3. Ein Handgerät (10) nach Anspruch 1 oder Anspruch 2, wobei besagtes Temperatureinstellelement (60) so angebracht ist, dass eine Temperatur von unter 25°C an besagtem einen Ende, das den Behandlungsbereich der Haut (100) berührt, entsteht.

4. Ein Handgerät (10) nach Anspruch 1, wobei besagte Steuer- und Treiberschaltung (120) im Betrieb besagtes Temperatureinstellelement (60) mit einer Folge von Impulsen beaufschlagt.

5. Ein Handgerät (10) nach Anspruch 1, wobei besagtes Temperatureinstellelement (60) ringförmig ist, und es sich bei besagter Öffnung (65) um eine zentrale Öffnung in besagter Ringform (60) handelt.

6. Ein Handgerät (10) nach Anspruch 1, wobei besagte mindestens eine Öffnung (65) eine Matrix von Öffnungen bzw. eine Vielzahl von schlitzförmigen Öffnungen beinhaltet.

7. Ein Handgerät (10) nach Anspruch 6, wobei besagte Vielzahl von Öffnungen so beabstandet ist, dass sich besagte gepulste Lichtenergie aus benachbarten Öffnungen innerhalb besagten Behandlungsbereichs der Haut (100) in einer vorbestimmten Epidermistiefe (115) überlappt.

8. Ein Handgerät (10) nach Anspruch 1, wobei besagte Folge von Impulsen besagter Lichtquelle (70) ausreichend Energie besitzt, um eine Fluenz von 4-25 J/cm² Lichtenergie von besagter Lichtquelle (70) an besagtem Teil des Behandlungsbereichs der Haut (110) über eine vorbestimmte Behandlungszeit zu liefern.

9. Ein Handgerät (10) nach Anspruch 8, wobei die vorbestimmte Behandlungszeit im Bereich von 5 - 60 Sekunden liegt.

10. Ein Handgerät (10) nach Anspruch 1, wobei jeder der besagten Impulse jener Folge von Impulsen ausreichend Energie besitzt, um eine Fluenz von 0,05 - 1 J/cm² Lichtenergie von besagter Lichtquelle (70) an besagtem Teil des Behandlungsbereichs der Haut (110) zu liefern.

11. Ein Handgerät (10) nach Anspruch 10, wobei jeder der besagten Impulse jener Folge von Impulsen ausreichend Energie besitzt, um eine Fluenz von 0,3 - 0,6 J/cm² Lichtenergie von besagter Lichtquelle (70) an besagtem Teil des Behandlungsbereichs der Haut (110) zu liefern.

12. Ein Handgerät (10) nach Anspruch 1, wobei besagte Lichtquelle (70) Licht mit einer Wellenlänge im Bereich von 300 - 2000 nm aussendet.

13. Ein Handgerät (10) nach Anspruch 1, wobei besagter Tel des Behandlungsbereichs der Haut (110) im Bereich von 0,25 - 2 cm² liegt.

14. Ein Handgerät (10) nach Anspruch 1, wobei besagte Folge von Impulsen besagter Lichtquelle eine Frequenz von 0,1 - 10 Hz und einen Tastgrad von höchstens 50% aufweist.

15. Ein Handgerät (10) nach Anspruch 1, wobei besagte Folge von Impulsen besagter Lichtquelle eine Frequenz von 0,25 - 5 Hz und einen Tastgrad von höchstens 50% aufweist.

## Revendications

1. Dispositif portatif (10) pour le traitement d'une zone à traiter sur la peau, le dispositif (10) comportant :
- un logement (20) présentant une ouverture ;
- un élément d'ajustement de la température (60), fixé à une extrémité dudit logement (20), l'une des extrémités dudit élément d'ajustement de la température (60) étant disposée de manière à rentrer en contact avec la zone à traiter sur la peau, ledit élément d'ajustement de la température (60) présentant au moins une ouverture (65) traversante ;
- une source de lumière (70) fixée sur ledit logement (20) ;
- un conduit à lumière (30) disposé de manière à laisser passer l'énergie lumineuse depuis ladite source de lumière (70) vers au moins une portion de la zone (110) à traiter sur la peau à travers ladite au moins une ouverture (65) ; et
- un circuit de contrôle et de pilotage (120) communiquant électriquement avec ladite source de lumière (70) et ledit élément d'ajustement de la température (60), ledit circuit de contrôle et de pilotage opérant afin d'alternativement :
émettre un train d'impulsions vers ladite source de lumière (70) fournissant ainsi de l'énergie lumineuse pulsée à ladite portion (110) de la zone à traiter sur la peau depuis ladite source de lumière (70) passant par ladite ouverture (65) ; et
mettre sous tension ledit élément d'ajustement de la température (60) afin d'ajuster la température de la zone (100) à traiter sur la peau.

2. Dispositif portatif (10) selon la revendication 1, dans lequel ledit élément d'ajustement de la température (60) est un élément thermoélectrique.

3. Dispositif portatif (10) selon la revendication 1 ou 2, dans lequel ledit élément d'ajustement de la température (60) est disposé de manière à fournir une température inférieure à 25°C à ladite extrémité disposée de manière à rentrer en contact avec la zone (100) à traiter sur la peau.

4. Dispositif portatif (10) selon la revendication 1, dans lequel ledit circuit de contrôle et de pilotage (120) opère afin de mettre sous tension ledit élément d'ajustement de la température (60) avec un train d'impulsions.

5. Dispositif portatif (10) selon la revendication 1, dans lequel ledit élément d'ajustement de la température (60) a une forme en anneau, et ladite ouverture (65) correspond à une ouverture centrale dans ladite forme en anneau (60).

6. Dispositif portatif (10) selon la revendication 1, dans lequel au moins une ouverture (65) comprend une matrice d'ouvertures et une pluralité d'ouvertures en fente.

7. Dispositif portatif (10) selon la revendication 6, dans lequel les ouvertures de ladite pluralité d'ouvertures sont espacées de telle sorte que ladite énergie lumineuse pulsée issue des ouvertures adjacentes se recouvre dans ladite zone (100) à traiter sur la peau à une profondeur épidermique (115) prédéterminée.

8. Dispositif portatif (10) selon la revendication 1, dans lequel les impulsions dudit train d'impulsions de ladite source de lumière (70) ont une énergie suffisante pour fournir une fluence comprise entre 4 et 25 J/cm² d'énergie lumineuse depuis ladite source de lumière (70) à ladite portion (110) de la zone à traiter sur la peau sur une durée de traitement prédéterminée.

9. Dispositif portatif (10) selon la revendication 8, dans lequel la durée prédéterminée de traitement est comprise entre 5 et 60 secondes.

10. Dispositif portatif (10) selon la revendication 1, dans lequel chacune desdites impulsions dudit train d'impulsions a une énergie suffisante pour fournir une fluence comprise entre 0.05 et 1 J/cm² d'énergie lumineuse depuis ladite source de lumière (70) à ladite portion (110) de zone à traiter sur la peau.

11. Dispositif portatif (10) selon la revendication 10, dans lequel chacune desdites impulsions dudit train d'impulsions a une énergie suffisante pour fournir une fluence comprise entre 0.3 et 0.6 J/cm² d'énergie lumineuse depuis ladite source de lumière (70) à ladite portion (110) de zone à traiter sur la peau.

12. Dispositif portatif (10) selon la revendication 1, dans lequel ladite source de lumière (70) émet de la lumière présentant des longueurs d'onde comprises entre 300 et 2000 nm.

13. Dispositif portatif (10) selon la revendication 1, dans lequel ladite portion (110) de zone à traiter sur la peau a une aire comprise entre 0.25 et 2 cm².

14. Dispositif portatif (10) selon la revendication 1, dans lequel ledit train d'impulsions de ladite source de lumière présente une fréquence comprise entre 0.1 et 10 Hz et un rapport cyclique inférieur à 50%.

15. Dispositif portatif (10) selon la revendication 1, dans lequel ledit train d'impulsions de ladite source de lumière présente une fréquence comprise entre 0.25 et 5 Hz et un rapport cyclique inférieur à 50%.
